Europäisches Patentamt

**European Patent Office**

Office européen des brevets

(11) Numéro de publication: **0 079 823**
**A1**

# DEMANDE DE BREVET EUROPEEN

(21) Numéro de dépôt: **82402037.4**

(22) Date de dépôt: **05.11.82**

(51) Int. Cl.³: **A 61 F 5/04**
**A 61 F 5/34, A 42 B 3/00**
**B 65 D 81/02**

(30) Priorité: **17.11.81 FR 8121444**

(43) Date de publication de la demande:
**25.05.83 Bulletin 83/21**

(84) Etats contractants désignés:
**AT BE CH DE GB IT LI LU NL SE**

(71) Demandeur: **Nataf, Paul**
**41, Boulevard Richard Wallace**
**F-92800 Puteaux(FR)**

(72) Inventeur: **Nataf, Paul**
**41, Boulevard Richard Wallace**
**F-92800 Puteaux(FR)**

(74) Mandataire: **Flechner, Willy et al,**
**CABINET FLECHNER 22, Avenue de Friedland**
**F-75008 Paris(FR)**

(54) **Dispositif de contention.**

(57) Dispositif de contention gonflable, utile notamment en médecine, comprenant un élément rigide (1) et une membrane (3) fixée à l'élément rigide (1) de manière à délimiter avec lui une cavité fermée, au moins deux orifices (5) en coïncidence étant ménagés l'un dans l'élément rigide, l'autre dans la membrane. Les deux orifices (5) communiquent l'un avec l'autre par un soufflet étanche qui s'étend dans la cavité.

./...

EP 0 079 823 A1

Croydon Printing Company Ltd.

FIG_1

Dispositif de contention.

La présente invention se rapporte à un nouveau type de matériels de contention qui, dans le domaine médical notamment, apporte avec une fabrication aisée et simplifiée, donc plus économique, une très grande facilité d'utilisation qui ouvre dans la pratique la voie à des applications multiples dépassant le domaine médical pour entrer dans celui de la protection en permettant la fabrication, entre autres, d'un nouveau type de casque protecteur, ou encore dans le domaine de certaines manipulations ou transports délicats, comme par exemple les tubes cathodiques utilisés en T.V., un nouveau type d'emballage pouvant être réutilisé, donc plus économique.

Tout le long de l'exposé, il sera pris comme exemple à l'appui de la présente invention, la contention d'une jambe, ce qui permettra de faire ressortir tous les avantages que l'on obtient en utilisant un matériel basé et conçu selon une conception fondamentalement différente, par rapport à ce qui existe déjà.

Certains dispositifs de contention, très simples mais généralement utilisés en relais, en attendant le plâtrage de la jambe, se présentent sous l!aspect d'un petit matelas gonflable à boudins, qui sert à envelopper la jambe et que l'on gonfle ensuite. Ce matelas permet la contention, mais sans plus, et est généralement utilisé à titre provisoire.

D'autres, plus sophistiqués (brevets français 71.00289 et 74.34584), sont prévus pour se substituer aux plâtres classiques, tout en permettant la respiration cutanée, ainsi que le massage d'un muscle sans nuire à la contention, ce qui évite son atrophie du muscle. Ils se composent de quatre éléments indépendants, soit : deux demi-coquilles et deux enveloppes souples ou membranes.

- Les deux demi-coquilles sont rigides ou semi-rigides de même conformation générale et capables de s'affronter par leurs bords respectifs, pour entourer sans la toucher, tout ou partie de la jambe traitée ; chaque demi-coquille est percée d'un certain nombre d'orifices identiques, avec en supplément deux autres pour permettre le passage de deux tubes pour le gonflage et massage rythmé.

- Les deux enveloppes souples sont gonflables. Il est aménagé sur l'une des parois de chacune d'elles deux orifices autour desquels et sur la face externe sont scellés respectivement un tube devant servir, entre autres, au gonflage. Sur les deux parois de chaque enveloppe, il est aménagé des orifices disposés en vis-à-vis, mais d'une façon devant permettre de se trouver positionnés ultérieurement en face de ceux des demi-coquilles. On procède au soudage des deux extrémités de tubes souples à soufflets autour de chacun des orifices en vis-à-vis, mais par les faces internes de chaque paroi, ce qui rend solidaire les deux parois de chaque enveloppe, on en termine la fabrication en soudant sur leur pourtour et entre eux les bords des parois; chaque enveloppe a autant de cheminées d'aération que de tubes souples à soufflets.

On procède à l'installation de chacune des enveloppes à l'intérieur en arc de cercle de chaque demi-coquille, en faisant passer les tubes de gonflage au travers des orifices aménagés à cet effet sur chaque demi-coquille, puis en faisant coïncider chaque cheminée d'aération avec l'orifice correspondant des demi-coquilles en tapissant le fond de chacune d'elles avec les deux parois de chacune des enveloppes, la paroi non en contact direct avec sa demi-coquille sera celle qui viendra s'appliquer sur la jambe, ce sera la face profonde.

Lorsque celle-ci est installée entre les deux demi-coquilles ainsi aménagées et après leur affrontement rendu permanent, on envoie par le tube approprié un fluide sous pression, l'enveloppe par la paroi déjà mentionnée viendra exercer une contention sur tous les points de la jambe. C'est du moins ce qui doit se passer théoriquement, il en va tout autrement dans la pratique.

Le soufflet aménagé sur le pourtour de l'enveloppe, pour donner de l'ampleur à la face profonde en mouvement sans tirer sur l'autre qui doit rester plaquée contre la demi-coquille par la pression exercée du fluide introduit, est gêné dans son effet par la ligne de rigidité que constitue le soudage périphérique des bords de l'enveloppe.

La face profonde de l'enveloppe forme, au repos, un arc de cercle d'un certain diamètre; en action sous l'impulsion du fluide sous pression introduit dans l'enveloppe, cette face profonde a tendance, et c'est le but escompté, à venir vers le centre, ce qui a pour conséquence de réduire le diamètre de son arc de cercle, tout en conservant sa surface de départ ; n'ayant aucune possibilité de se loger latéralement, cette surface en surplus se froisse et forme des faux-plis. Les plis lui sont dommageables, entraî-

nant de fréquents changements qui augmentent d'autant le coût d'utilisation, mais le défaut le plus important est celui que les faux-plis provoquent en marquant la peau de la jambe tout en risquant de l'irriter, et ce défaut devient rédhibitoire et même dangereux dans le cadre d'une utilisation d'un matériel de ce type, pour une contention faciale.

Enfin, la mise en oeuvre d'une enveloppe gonflable de ce type,dans laquelle on soude sur les faces destinées à en constituer l'intérieur des tubes souples à soufflets, présente de grandes difficultés techniques, un déchet de fabrication non négligeable et nécessite une main-d'oeuvre spécialisée et de haut niveau, de plus l'installation dans la demi-coquille doit se faire d'une façon minutieuse ; tous ces facteurs en alourdissent le prix de revient, sans en simplifier l'utilisation.

Les dispositifs hybrides et gonflables suivant l'invention de toutes formes, en matière plastique, apportent avec une conception originale la solution aux problèmes posés.

Le dispositif suivant l'invention gonflable comprend un élément rigide, une membrane fixée à l'élément rigide de manière à délimiter avec lui une cavité fermée, deux orifices ménagés l'un dans l'élément rigide, l'autre dans la membrane et un soufflet étanche qui s'étend dans la cavité et qui met les deux orifices en communication.

On peut procéder à la fabrication de ces dispositifs dans l'ordre suivant, et en vue de la contention d'une jambe ou d'une hauteur de jambe, on utilisera seulement deux éléments capables de s'affronter.

- Fabrication de deux demi-coquilles rigides ou semi-rigides aptes à résister à la pression d'un fluide sans se déformer selon les procédés de formage déjà connus, avec de chaque côté un méplat qui sera creusé latéralement pour créer un canal, lors de l'affrontement des demi-coquilles par leurs méplats respectifs en formant une coquille, deux canaux formeront un évent faisant communiquer l'intérieur de la coquille avec l'extérieur. On aménage sur chaque demi-coquille et, de préférence, sur son dôme une série d'orifices suffisamment espacés pour ne pas affaiblir celle-ci et disposés, de préférence, en ligne dans le sens longitudinal de chaque demi-coquille. On procède à l'aménagement de deux autres orifices autour desquels on scelle de préférence par soudage, et pour chacun d'eux, l'extrémité d'un embout bouchable à l'autre extrémité. On pourra ultérieurement utiliser indifféremment l'un des deux pour envoyer un fluide quelconque sous pression, et les deux pour effectuer soit une purge, soit un massage alterné.

- Découpage de deux membranes souples mais résistantes, qui peuvent être aussi bien en caoutchouc de type médical et stérilisable, cette découpe se fait en fonction de la conformation déterminée que prendra chacune des membranes quand elles seront solidarisées respectivement avec une demi-coquille. On procède ensuite à l'aménagement d'orifices identiques et de même disposition que ceux aménagés sur le dôme des demi-coquilles. Sur l'une des faces de chaque membrane, qui en deviendra la face interne, on procède au soudage, de préférence autour de chaque orifice, d'une des embases d'un tube souple à soufflets en position originelle rétractée, on procède au soudage de l'autre embase autour de chacun des orifices correspondants aménagés sur chaque demi-coquille, mais par sa face interne. On procède au soudage des deux extrémités longitudinales de chaque membrane sur la face interne et en arc de cercle de chaque demi-coquille et on soude enfin les côtés latéraux de chaque membrane sur la face frontale de chacun des méplats, en prenant bien soin de laisser libre chaque canal.

En associant ainsi irréversiblement et en les aménageant dans une forme, ici compatible pour la contention d'une jambe ou d'une hauteur de jambe, une paroi rigide ou semi-rigide avec une membrane souple, on obtient selon la présente invention une capacité ou cavité concave à forme et volume variable sous l'action d'un fluide quelconque sous pression. La position géométrique donnée ici à la membrane souple est, par rapport à l'arc de cercle de la demi-coquille, celle d'une corde d'arc légèrement détendue, et à partir du moment où l'on introduit dans la capacité un fluide quelconque sous pression, celui-ci n'exerce sa tension que sur la membrane souple, n'étant pas opérant sur la paroi de la demi-coquille, la membrane se transforme alors en arc de cercle avec sa face externe qui vient,dans sa plus grande partie, agir en contention sur la jambe et permet d'augmenter le volume de la capacité, dans son mouvement de transformation la membrane entraîne dans sa descente les tubes souples à soufflets qui ont un allongement modulé, en fonction de la conformation de la jambe. Ce procédé de contention ne provoque aucun faux-plis sur la surface de la membrane.

En effet, le surplus de surface disponible de la membrane s'aménage de lui-même dans la capacité qui a changé de forme et de volume, mais qui les reprendra quand le fluide sous pression sera évacué et qu'il n'y aura plus que celle de l'air ambiant, il en sera de même pour les tubes souples à soufflets. Enfin, les évents constitués au moment de l'affrontement des deux demi-coquilles par les méplats facilitent l'évacuation de l'air retenu au moment de la contention, entre la membrane et la peau de la jambe, éliminant ainsi les poches d'air.

Les exemples donnés et les figures explicatives à l'appui de la présente invention, et dont les proportions et les différents rapports d'épaisseur et/ou de dimension ne sont pas volontairement respectés pour une meilleure visualisation, ne sont pas limitatifs et peuvent faire l'objet de très nombreuses variantes, aménagements, combinaisons et applications ne sortant pas du cadre de la présente invention ; on pourra, notamment dans le domaine médical, utiliser unitairement ou non des matériels hybrides et gonflables de toutes formes, identiques ou non identiques, en fonction de la conformité de la partie du corps, des membres ou de la tête à traiter et/ou à protéger.

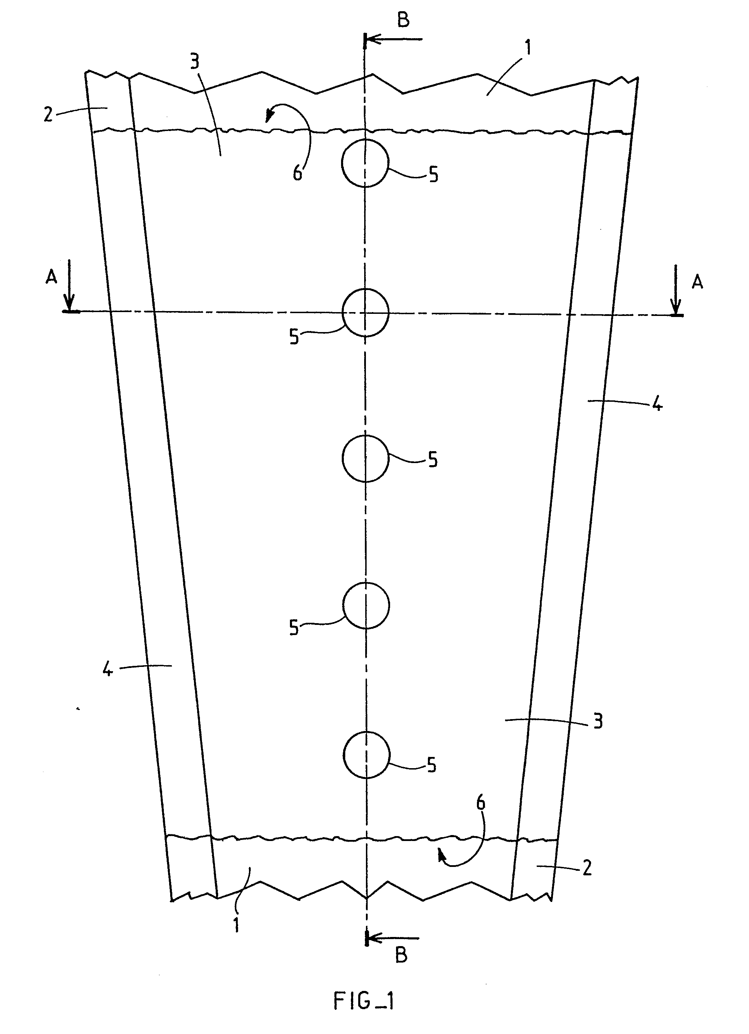

La figure 1 est une vue, en coupe verticale et sur un plan frontal, d'une fraction de la demi-coquille et de sa membrane, avec les orifices servant de cheminées d'aération. ainsi qu'une fraction des deux extrémités de la capacité.

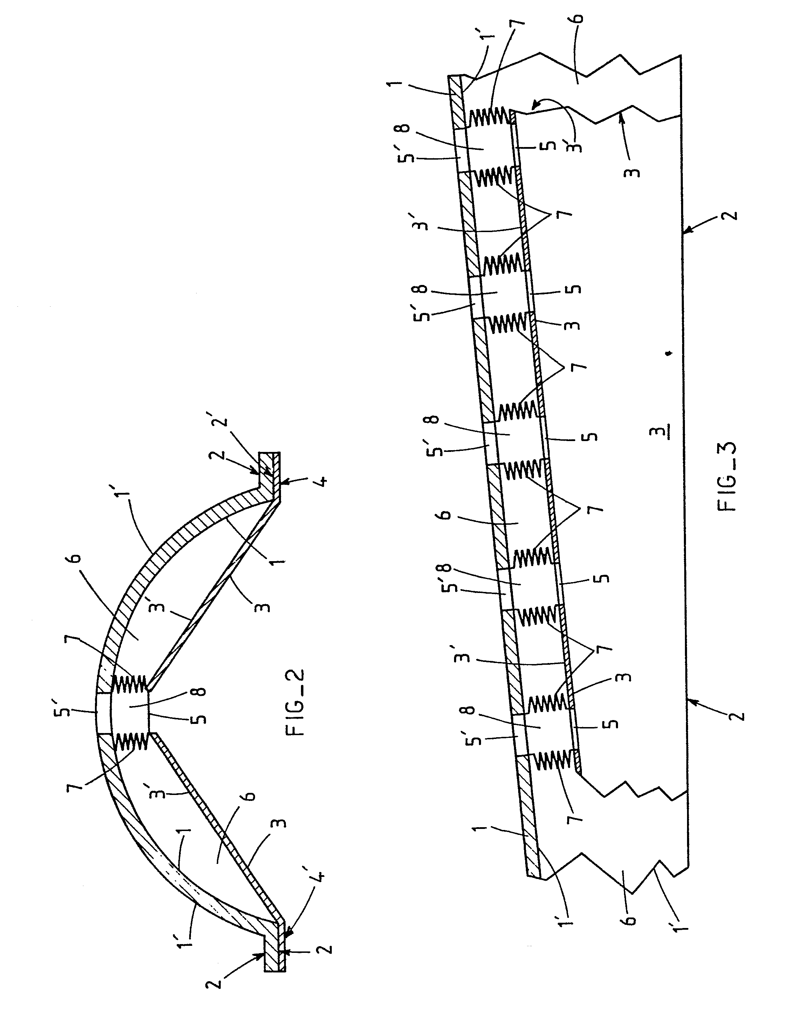

La figure 2 est une vue en coupe, selon la ligne A-A de la figure 1, de la demi-coquille, de la membrane souple avec un de ses tubes à soufflets en position originelle rétractée et dont l'autre extrémité est soudée à la paroi interne de la demi-coquille, l'ensemble formant une capacité concave. Par souci de simplification. aucun des tubes de gonflage situés sur la face externe de la demi-coquille n'a été représenté.

La figure 3 est une vue en coupe selon la ligne B-B de la figure 1, qui montre une fraction de la membrane et les tubes souples à soufflets formant autant de cheminées d'aération. La demi-coquille en coupe est montrée reposant sur l'autre méplat dont on ne peut voir que la ligne, également une fraction de la capacité.

Selon la figure 1, on distingue la face interne 1 de la demi-coquille, avec sa face frontale 2 des méplats, la face externe 3 de la membrane souple et ses bords latéraux 4 soudés par l'autre face sur les faces frontales 2 des méplats. Sur la face externe 3 de la membrane souple sont aménagés les orifices 5 des cheminées d'aération. La cavité fermée constituée entre la face interne 1 de la demi-coquille et la face interne non visible de la membrane souple est indiquée par la flèche directionnelle 6.

Selon la figure 2, on distingue la demi-coquille supérieure avec sa face interne 1 et externe 1', les deux méplats avec leurs faces frontales 2 et externe 2', les bords latéraux 4 de la membrane souple dont la face interne 4' est soudée sur les faces frontales 2 des méplats, entre l'orifice 5 aménagée dans la membrane souple et l'orifice 5' aménagé dans la paroi de la demi-coquille, et autour d'eux est soudé un soufflet 7 qui constitue la cheminée d'aération, ce soufflet est soudé par les faces internes de la demi-coquille et de la membrane, respectivement référencées 1 et 3'.

Selon la figure 3, on distingue la moitié de la demi-coquille partagée dans le sens longitudinal, on en distingue la face interne 1 et sa face externe 1' reposant sur un méplat représenté en 2 par une simple ligne, les soufflets 7 relient par les faces internes 1 de la demi-coquille et 3' de la membrane en étant soudés autour des orifices 5 et 5' et qui constituent autant de cheminées d'aération 8, la capacité constituée est indiquée par les flèches directionnelles 6.

Quand le dispositif est utilisé comme emballage de protection contre les chocs, les soufflets jouent aussi le rôle d'amortisseurs.

De préférence, les extrémités des coquilles sont recourbées de manière à limiter l'expansion de la membrane hors de la coquille.

REVENDICATIONS

1. Dispositif de contention gonflable comprenant un élément rigide (1) et une membrane (3) fixée à l'élément rigide (1) de manière à délimiter avec lui une cavité fermée, au moins deux orifices (5, 5') en coïncidence étant ménagés l'un dans l'élément rigide, l'autre dans la membrane, caractérisé en ce que les deux orifices (5, 5') communiquent l'un avec l'autre par un soufflet (7) étanche qui s'étend dans la cavité.

2. Dispositif suivant la revendication 1, caractérisé en ce que l'agencement de la membrane (3) et du soufflet (7) est tel que la tension de la membrane (3) provoque l'allongement du soufflet (7).

3. Dispositif suivant la revendication 1, caractérisé en ce qu'il affecte la forme d'un dispositif de protection d'une partie du corps humain.

4. Dispositif suivant la revendication 1, caractérisé en ce qu'il affecte la forme d'un casque de protection.

5. Dispositif suivant la revendication 1, caractérisé en ce qu'il affecte la forme d'un emballage.

0079823

FIG _1

0079823

FIG_2

FIG_3

Office européen
des brevets

**RAPPORT DE RECHERCHE EUROPEENNE**

**0079823**
Numéro de la demande

EP   82  40  2037

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | GB-A-    2 995   (HUMPHRIS)(AD 1910)<br>*Page 2, ligne 30 - page 3, ligne 18; figures* | 1 | A 61 F    5/04<br>A 61 F    5/34<br>A 42 B    3/00<br>B 65 D   81/02 |
| | --- | | |
| A | US-A-2 618 780   (CUSHMAN)<br>*Colonne 1, ligne 60 - colonne 3, ligne 36; figures 1,2,4,6* | 1,3,4 | |
| | --- | | |
| A | GB-A-1 355 243   (LARSON)<br>*Page 2, lignes 70-95; page 2, ligne 125 - page 3, ligne 9; page 3, lignes 63-69; figures 1,2,9* | 1,3 | |
| | --- | | |
| A | FR-A-2 321 266   (GUISET)<br>*Page 6, ligne 38 - page 7, ligne 31; figures 2-4* | 1 | |
| | | | DOMAINES TECHNIQUES RECHERCHES (Int. Cl. 3) |
| | --- | | |
| A | FR-A-1 255 906   (MOHIER)<br>*Page 2, colonne de droite, ligne 24 - page 3, colonne de droite, ligne 40; figures 5-8* | 1,5 | A 61 F<br>A 61 H<br>A 42 B<br>B 65 D |
| | --- | | |
| A | FR-A-1 006 109   (BENDER)<br>*Page 1, colonne de droite, ligne 40 - page 2, colonne de gauche, ligne 23; figures 1,2* | 1 | |
| | --- | | |
| A | WO-A-8 102 517   (SCHMID)<br>*Page 5, ligne 9 - page 6, ligne 21; figures 1-3* | 1 | |
| | ----- | | |

Le présent rapport de recherche a été établi pour toutes les revendications

| Lieu de la recherche<br>LA HAYE | Date d'achèvement de la recherche<br>16-03-1983 | Examinateur<br>BARTLETT S.C |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82